# EUROPÄISCHE PATENTSCHRIFT

(11) **EP 0 803 258 B1**
(45) Veröffentlichungstag und Bekanntmachung des Hinweises auf die Patenterteilung: **06.11.2002**
(21) Anmeldenummer: 97104032.4
(22) Anmeldetag: 11.03.1997
(51) Int. Cl.: A61M 1/00, F04B 45/02, F04B 33/00

(54) **Absaugpumpe**
Suction pump
Pompe d'aspiration

(30) Priorität: 27.04.1996 DE 19616954
(43) Veröffentlichungstag der Anmeldung: 29.10.1997
(73) Patentinhaber: wero-medical Werner Michallik GmbH & Co. KG, 65232 Taunusstein (DE)
(72) Erfinder: Marka, Rudolf, Dr., 64287 Darmstadt (DE); Maier, Stefan, 64331 Weiterstadt (DE); Peuker, Matthias, 63457 Hanau (DE); Kühn, Hans-Joachim, 65197 Wiesbaden (DE); Scheller, Walter, 63477 Maintal (DE)
(74) Vertreter: Weber, Dieter, Dr.

(56) Entgegenhaltungen:
- EP-A- 0 245 876
- EP-A- 0 271 620
- US-A- 5 304 129

## Beschreibung

Die Erfindung betrifft eine Absaugpumpe insbesondere für medizinische Zwecke mit einem Aufnahmebehälter mit zwei nebeneinander angeordneten Kammern, die mit einem Absaugschlauch verbunden sind und an denen jeweils ein etwa parallel zueinander bewegliches, einen Hohlraum teilweise umgebendes Element zur Erzeugung eines Vakuums angeordnet ist, wobei die beiden beweglichen Elemente über einen Hebel miteinander verbunden sind, dessen beiden Endteile am oberen Bereich jeweils eines beweglichen Elementes gehaltert sind und dessen Mittelteil in einer zwischen den Kammern angeordneten Lagerung beweglich gehaltert ist und wobei im oberen Bereich jedes beweglichen Elementes ein Einwege-Ventil angeordnet ist, welches öffnet, wenn der Druck in dem Hohlraum größer ist als der Umgebungsluftdruck.

Eine derartige Absaugpumpe ist beispielsweise aus EP 245 876 bekannt. Bei der hier beschriebenen Pumpe sind zwei Aufnahmebehälter über ein U-förmiges Verbindungsstück miteinander verbunden. Über jedem Behälter ist eine Vakuumpumpe angeordnet, deren bewegliches Pumpelement während der Pumpbewegung an den Seiten des Behälters entlanggleitet. Dadurch wird in dem von dem beweglichen Element und dem oberen Abschluß des Aufnahmebehälters gebildeten Raum ein Vakuum erzeugt, das über ein Ventil ein Vakuum in dem Aufnahmebehälter erzeugt und dadurch Sekret oder andere Flüssigkeit oder Erbrochenes aus beispielsweise dem Mund eines Patienten absaugt. Die beiden nebeneinander angeordneten beweglichen Pumpelemente sind über einen gemeinsamen Hebel miteinander verbunden, so daß bei jeder Hebelbewegung jeweils eines der beiden beweglichen Elemente ein Vakuum zum Einsaugen durch den Katheter erzeugt. Dadurch werden Totzeiten bei der Bedienung der Pumpe vermieden. Die Pumpe ist sehr funktionssicher, allerdings auch relativ aufwendig aufgebaut. Die nach jedem Absaugen erforderliche Reinigung der Pumpe erfordert eine recht aufwendige Demontage. Beide Aufnahmebehälter sind offen miteinander verbunden, so daß bei jeder Hubbewegung in einem relativ großen Volumen Vakuum durch einen relativ geringen Hub jeweils eines beweglichen Elementes erzeugt wird.

Aus EP 271 620 ist eine vollkommen anders geartete Absaugpumpe bekannt. Die hier beschriebene, mit der Hand zu betreibende Pumpe weist einen einzigen Hohlraum auf, in dem Vakuum erzeugt wird zur Absaugung von Körperflüssigkeit und ähnlichem. Die Handbedienung erfordert einen relativ hohen mechanischen Aufwand, beispielsweise durch den Einsatz von Rückstellfedern. Aufgrund der bei der Bedienung dieser Pumpe sich abwechselnden Ansaugzeiten und Totzeiten ist ein kontinuierliches Absaugen nicht möglich, so daß der Absaugprozeß entsprechend langsam verläuft. Außerdem wird durch die periodische Unterbrechung des Absaugvorganges ein Rückschlagventil am distalen Ende des Katheters notwendig. Darüber hinaus ist die Arbeit mit dieser Pumpe auf die Dauer recht ermüdend, da die zum Absaugen notwendige Arbeit ausschließlich durch Öffnen und Schließen der Hand der Bedienperson geleistet wird.

Aufgabe der vorliegenden Erfindung ist es, ausgehend von den Nachteilen des beschriebenen Standes der Technik, eine Absaugpumpe bereitzustellen, die sowohl leicht zu bedienen als auch leicht zu reinigen ist und eine sichere Absaugung ermöglicht.

Diese Aufgabe wird erfindungsgemäß dadurch gelöst, daß jeweils eine Kammer des Aufnahmebehälters und ein bewegliches Element einen gemeinsamen, variablen Hohlraum umschließen, wobei das bewegliche Element den oberen Bereich des Hohlraumes begrenzt und daß die beiden Hohlräume über ein gemeinsames Verteilerrohr mit einem Anschluß für den Absaugschlauch verbunden sind, wobei am Eingang eines jeden Hohlraumes in das Verteilerrohr ein weiteres Einwegeventil angeordnet ist, das schließt, wenn der Druck in dem Hohlraum größer ist als der Umgebungsdruck.

Zweckmäßige Weiterbildungen der Erfindung sind Gegenstand der abhängigen Ansprüche.

Eine derartige Absaugpumpe kommt mit einem Minimum an Einzelteilen aus, da der gesamte Hohlraum von einem einheitlichen Material begrenzt werden kann, wobei das bewegliche Element vorzugsweise als Faltenbalg ausgebildet ist. Der Behälter kann einfach und damit preiswert hergestellt werden und als Einwegbehälter ausgebildet sein, so daß eine Reinigung nach Gebrauch nicht erforderlich ist, da der Behälter dann mit samt seinem Inhalt entsorgt werden kann. Allerdings ist es auch möglich, den Behälter zu reinigen, insbesondere wenn er aus einem autoklavierbaren Material hergestellt ist. Dadurch, daß beide Hohlräume zwar nebeneinander angeordnet sind, aber keine offene Verbindung aufweisen, und sich dadurch in beiden Hohlräumen ein unterschiedlicher Druck ausbilden kann, wird Vakuum jeweils nur in einem der beiden benachbarten Hohlräume gebildet, das dann entsprechend kräftig ist. Durch abwechselnde Unterdruckerzeugung in den beiden Behältern ist ein kontinuierliches Absaugen möglich.

Der Hebel kann zweckmäßigerweise als Trittplatte ausgebildet sein, so daß durch eine Hin- und Herbewegung des Fußes einer Bedienperson auf dem Hebel das Vakuum alternierend in beiden Hohlräumen gebildet wird. Zweckmäßigerweise ist dabei der Hebel lösbar an den beweglichen Elementen gehaltert, um die Reinigung zu erleichtern bzw. eine Wiederverwendung des Hebels bei Entsorgung des als Einwegbehälter ausgebildeten Aufnahmebehälters zu ermöglichen.

Es ist zweckmäßig, daß der Aufnahmebehälter in einem Grundgehäuse gehaltert ist, das eine Bodenplatte und Seitenwände aufweist und daß das Grundgehäuse die Lagerung für den Hebel aufweist. Dadurch läßt sich eine besonders hohe Stabilität der Absaugpumpe erzielen.

Nachstehend wird ein Ausführungsbeispiel der Erfindung anhand einer Zeichnung näher erläutert.

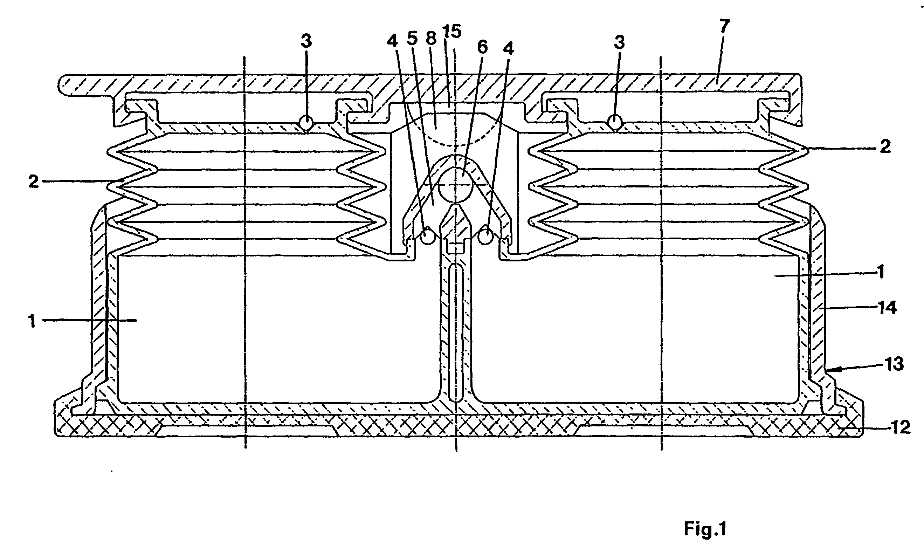

In der Zeichnung zeigt
- Figur 1: eine erfindungsgemäße Absaugpumpe im Querschnitt und
- Figur 2: eine Explosionsdarstellung der erfindungsgemäßen Absaugpumpe.

Die Absaugpumpe weist einen Aufnahmebehälter mit zwei nebeneinander angeordneten Kammern 1, deren Oberseite von jeweils einem beweglichen Element begrenzt wird. Das bewegliche Element 2 dient der Erzeugung des Vakuums in dem von dem Aufnahmebehälter 1 und dem beweglichen Element 2 gebildeten gemeinsamen Hohlraum. An der ebenen Oberseite des beweglichen Elementes 2, das als Faltenbalg ausgebildet ist, ist ein Einwegeventil 3 angeordnet, welches öffnet, wenn der Faltenbalg zusammengedrückt wird, so daß der durch das Zusammendrücken entstehende Überdruck im Inneren des Hohlraumes abgebaut wird. Während des Niederdrückens des einen Faltenbalges wird gleichzeitig der daneben angeordnete zweite Faltenbalg auseinandergezogen, so daß in dem entsprechenden Hohlraum ein Unterdruck entsteht. Durch diesen Unterdruck öffnet das zweite Einwegeventil 4, so daß in dem Verteilerrohr 5 und dem daran angeschlossenen Absaugschlauch 6 ein Vakuum gebildet wird und dadurch aus einer Körperhöhle, beispielsweise dem Mund eines Notfallpatienten, Sekret oder ähnliches abgesaugt werden kann.

Die Hub- bzw. Senkbewegung erfolgt durch den als Trittplatte ausgebildeten Hebel 7, der mit Lagerbolzen 15 in einer Lagerung 8 beweglich gehaltert ist. An der Unterseite des Hebels 7 sind Ausnehmungen 9 angeordnet, an denen der Hebel 7 auf entsprechende Gegenstücke 10 an den Oberseiten der beweglichen Elemente 2 aufgeschoben wird. Der Hebel 7 kann dadurch sehr leicht von den beweglichen Elementen 2 der Absaugpumpe abgenommen werden.

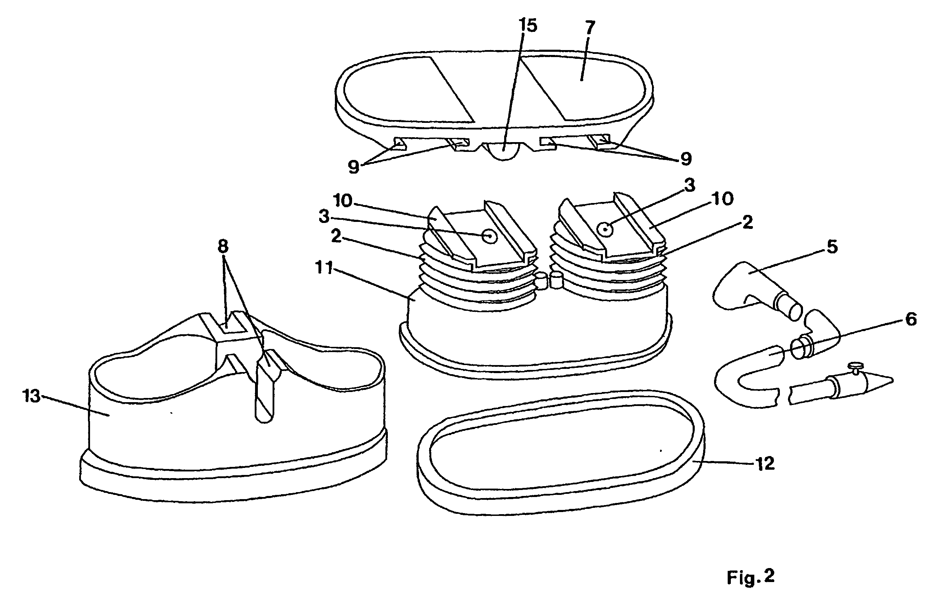

Der Aufnahmebehälter mit den Kammern 1 ist in einem Gehäuse 11 integriert (siehe Figur 2). Dieses Gehäuse 11 ist auf einer Bodenplatte 12 in dem Grundgehäuse 13 angeordnet und erhält dadurch eine hohe Stabilität. Die Verbindung von Bodenplatte 12 und Seitenwänden 14 des Grundgehäuses 13 mit dem Gehäuse 11 kann dabei durch Ineinanderschnappen der Ränder der einzelnen Teile erfolgen, wenn diese aus einem Material mit genügender Flexibilität, wie beispielsweise einem elastischen Kunststoff gebildet sind. Der Hebel 7 ist dabei in der in dem Grundgehäuse 13 angeordneten Lagerung 8 gehaltert.

Eine derartige Absaugpumpe ist sehr leicht zu montieren. Bei Ausbildung des gemeinsamen Hohlraumes bzw. des Gehäuses 11 mit den beiden Hohlräumen als Einwegbehälter kann diese Einheit leicht aus dem Grundgehäuse 13 entnommen und entsorgt werden, während das Grundgehäuse 13 und der Hebel 7 leicht gereinigt und wiederverwendet werden können.

## Patentansprüche

1. Absaugpumpe, insbesondere für medizinische Zwecke mit einem Aufnahmebehälter mit zwei nebeneinander angeordneten Kammern (1); die mit einem Absaugschlauch (6) Verbunden sind und an denen jeweils ein etwa parallel zueinander bewegliches, einen Hohlraum teilweise umgebendes Element (2) zur Erzeugung eines Vakuums angeordnet ist, wobei die beiden beweglichen Elemente (2) über einen Hebel (7) miteinander verbunden sind, dessen beiden Endteile am oberen Bereich jeweils eines beweglichen Elementes (2) gehaltert sind und dessen Mittelteil in einer zwischen den Kammern (1) angeordneten Lagerung (8) beweglich gehaltert ist und wobei im oberen Bereich jedes beweglichen Elementes (2) ein Einwege-Ventil (3) angeordnet ist, welches öffnet, wenn der Druck in dem Hohlraum größer ist als der Umgebungsluftdruck, **dadurch gekennzeichnet, daß** jeweils eine Kammer (1) und ein bewegliches Element (2) einen gemeinsamen, variablen Hohlraum umschließen, wobei das bewegliche Element (2) den oberen Bereich des Hohlraumes begrenzt und daß die beiden Hohlräume über ein gemeinsames Verteilerrohr (5) mit einem Anschluß für den Absaugschlauch (6) verbunden sind, wobei am Eingang eines jeden Hohlraumes in das Verteilerrohr (5) ein weiteres Einwege-Ventil (4) angeordnet ist, das schließt, wenn der Druck in dem Hohlraum größer ist als der Umgebungsdruck.

2. Absaugpumpe nach Anspruch 1, **dadurch gekennzeichnet, daß** der Hebel (7) als Trittplatte ausgebildet ist.

3. Absaugpumpe nach Anspruch 1 oder 2, **dadurch gekennzeichnet, daß** der Hebel (7) lösbar an den beweglichen Elementen (2) gehaltert ist.

4. Absaugpumpe nach einem der Ansprüche 1 bis 3, **dadurch gekennzeichnet, daß** der Aufnahmebehälter mit den Kammern (1) als Einweg-Behälter ausgebildet ist.

5. Absaugpumpe nach einem der Ansprüche 1 bis 4, **dadurch gekennzeichnet, daß** die Aufnahmebehälter mit den Kammern (1) in einem gemeinsamen Grundgehäuse (13), das eine Bodenplatte (12) und Seitenwände (14) aufweist, gehaltert sind und daß das Grundgehäuse (13) die Lagerung (8) für den Hebel (7) aufweist.

6. Absaugpumpe nach einem der Ansprüche 1 bis 5, **dadurch gekennzeichnet, daß** die beweglichen Elemente (2) als Faltenbalg ausgebildet sind.

## Claims

1. A suction removal pump, in particular for medical purposes, comprising a receiving container with two chambers (1) which are arranged side-by-side and which are connected to a suction removal hose (6) and at each of which is arranged a respective element (2) movable in substantially mutually parallel relationship and partially surrounding a hollow space, for producing a vacuum, wherein the two movable elements (2) are connected together by way of a lever (7) whose two end portions are mounted to the upper region of a respective movable element (2) and whose central portion is mounted movably in a mounting (8) arranged between the chambers (1) and wherein arranged in the upper region of each movable element (2) is a one-way valve (3) which opens when the pressure in the hollow space is greater than the ambient air pressure, **characterised in that** a chamber (1) and a movable element (2) respectively enclose a common variable hollow space, wherein the movable element (2) delimits the upper region of the hollow space and that the two hollow spaces are connected by way of a common distributor pipe (5) with a connection for the suction removal hose (6), wherein arranged at the entry of each hollow space into the distributor pipe (5) is a further one-way valve (4) which closes when the pressure in the hollow space is higher than the ambient pressure.

2. A suction removal pump according to claim 1 **characterised in that** the lever (7) is in the form of a foot plate.

3. A suction removal pump according to claim 1 or claim 2 **characterised in that** the lever (7) is mounted releasably to the movable elements (2).

4. A suction removal pump according to one of claims 1 to 3 **characterised in that** the receiving container with the chambers (1) is in the form of a disposable container.

5. A suction removal pump according to one of claims 1 to 4 **characterised in that** the receiving containers with the chambers (1) are mounted in a common main housing (13) which has a bottom plate (12) and side walls (14) and that the main housing (13) has the mounting (8) for the lever (7).

6. A suction removal pump according to one of claims 1 to 5 **characterised in that** the movable elements (2) are in the form of a bellows.

## Revendications

1. Pompe d'aspiration, notamment destinée à des fins médicales, comportant un récipient de collecte avec deux compartiments disposés côte à côte, qui sont reliés à un tuyau d'aspiration et sur chacun desquels est disposé un élément pour engendrer un vide, qui entoure partiellement une cavité, et qui est mobile à peu près parallèlement à l'autre élément, les deux éléments mobiles étant reliés l'un à l'autre par l'intermédiaire d'un levier dont les deux parties d'extrémité sont supportées chacune au niveau de la zone supérieure d'un élément mobile respectif, et dont la partie centrale est supportée de manière mobile dans un système de palier disposé entre les compartiments, et une soupape unidirectionnelle étant disposée dans la zone supérieure de chacun des éléments mobiles et s'ouvrant lorsque la pression dans la cavité est supérieure à la pression d'air environnante, **caractérisée en ce qu'**un compartiment (1) et un élément mobile (2) respectifs enferment une cavité commune, variable, l'élément mobile (2) délimitant la zone supérieure de la cavité, et **en ce que** les deux cavités sont reliées, par l'intermédiaire d'un tube distributeur commun (5), à un raccord de branchement pour le tuyau d'aspiration (6), une autre soupape unidirectionnelle (4) étant disposée à l'entrée de chaque cavité dans le tube distributeur (5), cette autre soupape unidirectionnelle se fermant lorsque la pression dans la cavité est supérieure à la pression environnante.

2. Pompe d'aspiration selon la revendication 1, **caractérisée en ce que** le levier (7) est réalisé sous forme d'une pédale.

3. Pompe d'aspiration selon la revendication 1 ou 2, **caractérisée en ce que** le levier (7) est supporté de manière détachable sur les éléments mobiles (2).

4. Pompe d'aspiration selon l'une des revendications 1 à 3, **caractérisée en ce que** le récipient de collecte, avec les compartiments (1), est réalisé sous forme d'un récipient à usage unique.

5. Pompe d'aspiration selon l'une des revendications 1 à 4, **caractérisée en ce que** les récipients de collecte, avec les compartiments (1), sont supportés dans un bâti de base commun (13), qui comporte une plaque de fond (12) et des parois latérales (14), et **en ce que** le bâti de base (13) présente le système de palier (8) pour le levier (7).

6. Pompe d'aspiration selon l'une des revendications 1 à 5, **caractérisée en ce que** les éléments mobiles (2) sont réalisés sous forme de soufflet.
